**Europäisches Patentamt**

(19) **European Patent Office**

**Office européen des brevets**

(11) Publication number : **0 421 322 B1**

# EUROPEAN PATENT SPECIFICATION

(45) Date of publication of patent specification :
05.01.94 Bulletin 94/01

(51) Int. Cl.$^5$ : **C07C 19/08, C07C 17/26**

(21) Application number : **90118809.4**

(22) Date of filing : **01.10.90**

(54) Process for preparing penta-fluorodichloropropanes.

(30) Priority : **30.09.89 JP 256608/89**
**30.09.89 JP 256609/89**
**26.01.90 JP 16380/90**

(43) Date of publication of application :
**10.04.91 Bulletin 91/15**

(45) Publication of the grant of the patent :
**05.01.94 Bulletin 94/01**

(84) Designated Contracting States :
**DE FR GB IT**

(56) References cited :
**GB-A- 1 010 352**
**COLLECTION OF CZECHOSLOVAK CHEM-**
**ICAL COMMUNICATION, vol. 36, 1971, pages**
**1867-1875, Prague, CS; O. PALETA et al.:**
**"Addition reactions of haloolefins. XI. Reac-**
**tion of tetrafluoroethylene with monof-**
**luoromethanes in the presence of aluminum**
**chloride"**

(73) Proprietor : **DAIKIN INDUSTRIES, LIMITED**
**Umeda Center Building 4-12 Nakazaki-nishi**
**2-chome Kita-ku**
**Osaka 530 (JP)**

(72) Inventor : **Aoyama, Hirokazu**
**28-1-707, Nyoze-cho**
**Takatsuki-shi, Osaka-fu (JP)**
Inventor : **Yasuhara, Takashi**
**1-18-505, Asahigaoka**
**Toyonaka-shi, Osaka-fu (JP)**
Inventor : **Kono, Satoru**
**H-203, 17, Shin Ashiya Kami**
**Suita-shi, Osaka-fu (JP)**
Inventor : **Koyama, Satoshi**
**4-11-32-503, Zuiko, Higashiyodogawa-ku**
**Osaka-shi, Osaka-fu (JP)**

(74) Representative : **Hansen, Bernd, Dr.**
**Dipl.-Chem. et al**
**Hoffmann, Eitle & Partner Patent- und**
**Rechtsanwälte, Postfach 81 04 20**
**D-81904 München (DE)**

Jouve, 18, rue Saint-Denis, 75001 PARIS

## Description

The present invention relates to a process for preparing pentafluorodichloropropanes, namely 1,1,1,2,2-pentafluoro-3,3-dichloropropane (hereinafter referred to as "R-225ca") and 1,1,2,2,3-pentafluoro-1,3-dichloropropane (hereinafter referred to as "R-225cb"), which can be used as substitutes for industrially important 1,1,2-trichloro-1,2,2-trifluoroethane and have less influence to environments that 1,1,2-trichloro-1,2,2-trifluoroethane.

Hitherto, as a commercial process for preparing R-225ca and R-225cb, there is known a process comprising reacting tetrafluoroethylene (hereinafter referred to as "TFE") with fluorodichloromethane (hereinafter referred to as "R-21") at a temperature of from 15 to 100°C in the presence of a catalyst such as anhydrous aluminum chloride (cf. U.S. Patent No. 2,462,402, J. Am. Chem. Soc., 71 (1949) 979, Collec. Czec. Chem. Com., 36 (1971) 1867). As a process for preparing R-225ca, there is known a process comprising reacting TFE with cesium fluoride in glyme and then with chloroform (cf. U.S. Patent No. 3,381,042).

In the former process, the reaction is batchwise carried out in an autoclave or a glass reactor at a temperature of from 15 to 100°C by using anhydrous aluminum chloride, and selectivities and yields of the desired products are low, for example, the yields are from 46 to 58 %. Therefore, the former process is uneconomical in the commercial production of R-225ca and R-225cb. In addition, to separate the reaction products from the catalyst after the reaction is completed, the reaction products should be collected with a cooled trap under reduced pressure or the catalyst is treated with dilute hydrochloric acid and then the reaction products are recovered.

In the latter process which utilizes cesium fluoride as one of the starting materials, a selectivity and an yield of R-225ca are good. However, cesium fluoride is extremely expensive, so that this process is not commercially attractive.

The above conventional processes should be carried out batchwise in a liquid phase, and the starting materials are reacted in a molar ratio of 1:1.

One object of the present invention is to provide a catalyst which catalyzes the reaction of TFE with R-21 to produce R-225ca and R-225cb in good selectivities and yields, and separation of which is not necessary after the reaction.

Another object of the present invention is to provide a process for producing R-225ca and R-225cb in good selectivities and yields.

According to the present invention, there is provided a process for preparing R-225ca and R-225cb which comprises reacting TFE with R-21 in the presence of a catalyst selected from the group consisting of an aluminum chlorofluoride of the formula:

$$AlCl_xF_yO_z$$

wherein x, y and z are numbers which satisfy the equations:

$$x + y + 2z = 3, 0 < x < 3, 0 \leq y < 3 \text{ and } 0 < z < 3/2$$

and anhydrous aluminum chloride provided that when the catalyst is anhydrous aluminum chloride, at least 2 moles of TFE is reacted with one mole of R-21 at a temperature of -20°C to +100°C.

In a preferred embodiment of the process of the present invention, a quantity of the catalyst is filled in a reactor tube, and then a mixture of TFE and R-21 is passed through the reactor tube, preferably in a gas phase, at a suitable temperature. A reaction mixture recovered from the exit of the reactor is purified by a per se conventional method such as rectification to obtain R-225ca and R-225cb. The reaction mixture recovered from the exit contains R-225ca and R-225cb in a molar ratio of 20:80 to 80:20.

The aluminum chlorofluoride may be prepared by reacting activated alumina with a chlorohydrocarbon, a chlorofluorohydrocarbon, a fluorohydrocarbon, hydrogen chloride or mixtures thereof at an elevated temperature.

For example, a quantity of alumina is filled in a reactor tube made of stainless steel, Hastelloy or glass and heated at a temperature of 300 to 500°C with flowing dry nitrogen to thoroughly dry alumina. Then, dried alumina is treated with the chlorohydrocarbon, the chlorofluorohydrocarbon or the fluorohydrocarbon or a mixture of the halohydrocarbon with hydrogen chloride or oxygen at a temperature of from 100 to 600°C, preferably from 200 to 400°C for a desired time. When the treating temperature is lower than 100°C, the treating time becomes long. When the treating temperature is higher than 700°C, carbon tends to deposit on surfaces of alumina particles so that a catalytic activity is deteriorated. Deterioration of the catalytic activity due to the deposited carbon can be prevented by the presence of oxygen or air (cf. Japanese Patent Publication No. 27375/1986).

When the alumina is treated with hydrogen chloride gas, it is firstly dried by heating it at a temperature of 400 to 800°C with flowing dry nitrogen. Then, the dried alumina is treated with hydrogen chloride at a temperature of from 300 to 700°C for 3 to 10 hours. Hydrogen chloride may be diluted with an inert gas such as nitrogen or argon or a chlorofluorohydrocarbon such as dichlorodifluoromethane (R-12) or R-21.

The alumina may be any of porous aluminas which are commercially available for dehydration or as a catalyst and comprise γ-alumina. Such alumina is commercially available under trade names of Neobead C, MHB, GB and D (from Mizusawa Chemical Co., Ltd.), activated alumina KHA, NKHI and NKH 3 (from Sumitomo Chemical Co., Ltd.).

As the chlorohydrocarbon or chlorofluorohydrocarbon which contains no hydrogen atoms, those having 1 to 3 carbon atoms, preferably 1 or 2 carbon atoms may be used. In particular, carbon tetrachloride, fluorotrichloromethane, difluorodichloromethane, trifluorochloromethane, 1,1,2-trichloro-1,2,2-trifluoroethane, 1,1,1-trichloro-2,2,2-trifluoroethane, 1,1,2,2-tetrafluoro-1,2-dichloroethane, 1,1,1,2-tetrafluoro-2,2-dichloroethane, 1,1,2,2-tetrachloro-1,2-difluoroethane, 1,1,1,2-tetrachloro-2,2-difluoroethane and the like are used.

As the chlorohydrocarbon or chlorofluorohydrocarbon containing one or more hydrocarbons, those having 1 to 3 carbon atoms, preferably 1 or 2 carbon atoms are used. In particular, fluorodichloromethane, difluorodichloromethane, 1,1,1-trifluoro-2,2-dichloroethane, 1,1,2-trifluoro-1,2-dichloroethane, 1,1,1-trifluoro-2-chloroethane and the like are sued.

Alternatively, the aluminum chlorofluoride may be prepared by treating the alumina with an inorganic fluoride at a specific temperature, for example, with hydrogen fluoride at a temperature of 20 to 450°C, with sulfur fluoride (e.g. $SF_4$ or $SF_6$), sulfuryl fluoride or thionyl fluoride at a temperature of 300 to 500°C, or with an ammonium fluoride (e.g. acidic ammonium fluoride or neutral ammonium fluoride) at a temperature of 20 to 450°C, and then with the chlorofluorohydrocarbon or chlorohydrocarbon.

Anhydrous aluminum chloride may be a commercially available particle-form or ground one.

In the process of the present invention, a molar ratio of R-21 to TFE is at least 1:2, preferably from 1:2 to 1:10. This molar ratio should be kept when anhydrous aluminum chloride is used as the catalyst.

When the molar ratio is less than 1:2, R-21 may be converted to R-22 (difluorochloromethane), R-23 (trifluoromethane) and/or chloroform in a larger amount. In the molar ratio range larger than 1:2, a conversion and a selectivity do not greatly change. As the ratio of TFE increases, an amount of TFE to be recycled increases. This is not economical. Then, the upper limit of the molar ratio is preferably 1:10.

When the process of the present invention is carried out in a gas phase, the raw materials may be diluted with an inert gas such as nitrogen or argon or one of the product compounds of the process, although the raw materials may be supplied as such.

A reaction pressure is not critical. Preferably, the reaction pressure is not lower than atmospheric pressure, since under reduced pressure, an apparatus becomes complicated. The upper limit of the pressure is a pressure at which the products are not liquefied.

The reaction according to the present invention is usually carried out in a reactor tube made of, for example, stainless steel or Hastelloy. When the reaction is carried out under atmospheric pressure, a glass tube can be used.

When the aluminum chlorofluoride is used as the catalyst, a reaction temperature is usually from -20 to +150°C, preferably from -20 to +100°C. When anhydrous aluminum chloride is used as the catalyst, the reaction temperature is from -20 to +100°C, preferably from -20 to +50°C. When the reaction temperature is higher than the above upper limit, amounts of by-products increases and the selectivities of R-225ca and R-225cb decreases.

The process of the present invention may be carried out in a liquid phase. To supply the raw materials in the liquid phase, TFE should be liquefied in the reaction system. Therefore, the reaction should be carried out under pressure.

The present invention will be illustrated by the following Examples.


Example 1


In a reactor tube having an inner diameter of 20 mm made of stainless steel SUS 316, Neobead GB (40 ml) was filled and dried by flowing dry nitrogen at 400°C for 6 hours. After the internal temperature was lowered to 300°C and the supply of nitrogen was stopped, R-21 was flowed in the tube at a flow rate of 75 ml/min. The exit gas was analyzed with gas chromatography. The supply of R-21 was continued till an amount of carbon dioxide in the exit gas did not decrease further. Thereafter, the tube was cooled, and a composition of the catalyst was analyzed according to an analogous method to the thermal hydrolysis method disclosed by P. Woodbridge et al in Nature, 229,626 (1971).

When the internal temperature reached 30°C, R-21 and TFE were flowed at flow rates of 20 ml/min. and 60 ml/min., respectively (a molar ratio of 1:3). After 3 hours, the exit gas was analyzed with gas chromatography. The results are shown in Table 1, in which R-224 represents dichlorotetrafluoropropane.

The compositions of the catalysts prepared in this Example and also in Examples 2-13 are summarized in Table 2.

## Example 2

In the same manner as in Example 1 but using Neobead C in place of Neobead GB and R-22 (difluorochloromethane) in place of R-21, the catalyst was prepared and the reaction was carried out. The results are shown in Table 1.

## Example 3

In the same manner as in Example 1 but using R-12 (dichlorodifluoromethane) in place of R-21, the catalyst was prepared and the reaction was carried out. The results are shown in Table 1.

## Example 4

In the same manner as in Example 1 but using R-114 (tetrafluorodichloroethane) in place of R-21, the catalyst was prepared and the reaction was carried out. The results are shown in Table 1.

## Example 5

In the same manner as in Example 1 but using carbon tetrachloride in place of R-21, the catalyst was prepared and the reaction was carried out. The results are shown in Table 1.

## Example 6

In the same manner as in Example 1 but using activated alumina NKH 3 (manufactured by Sumitomo Chemical Co., Ltd.) in place of Neobead GB, the catalyst was prepared and the reaction was carried out. The results are shown in Table 1.

## Example 7

In the same manner as in Example 1 but using activated alumina KHA (manufactured by Sumitomo Chemical Co., Ltd.) in place of Neobead GB, the catalyst was prepared and the reaction was carried out. The results are shown in Table 1.

## Example 8

In the same manner as in Example 1 but reacting R-21 and TFE at -20°C, the reaction was carried out. The results are shown in Table 1.

## Example 9

In the same manner as in Example 1 but reacting R-21 and TFE at 80°C, the reaction was carried out. The results are shown in Table 1.

## Example 10

In the same manner as in Example 1 but changing a molar ratio of R-21 to TFE from 1:3 to 1:10, the reaction was carried out. The results are shown in Table 1.

## Example 11

In the same manner as in Example 1 but changing a molar ratio of R-21 to TFE from 1:3 to 1:1, the reaction was carried out. The results are shown in Table 1.

## Example 12

In the same manner as in Example 1 but reacting R-21 and TFE at 160°C, the reaction was carried out.

The results are shown in Table 1.

Example 13

In the same manner as in Example 1 but reacting R-21 and TFE at 200°C, the reaction was carried out. The results are shown in Table 1.

Example 14

In a reactor tube having an inner diameter of 20 mm made of Hastelloy C, activated alumina NKH 3 (manufactured by Sumitomo Chemical Co., Ltd.) (20 ml) was filled and dried by flowing dry nitrogen at 400°C for 6 hours. After the internal temperature was lowered to 300°C, nitrogen and dry hydrogen chloride gas were flowed in the tube at flow rates of 50 ml/min. and 100 ml/min. After 3 hours, the heating was stopped and the material was cooled with flowing nitrogen to terminate the preparation of a catalyst.

The prepared catalyst (2.5 ml) was filled in a glass tube reactor having an inner diameter of 8 mm. Then, R-21 and TFE were flowed at flow rates of 10 ml/min. and 60 ml/min., respectively (a molar ratio of 1:6) after mixing them at an entrance of the reactor tube. During the reaction, the tube was heated from outside so that the internal temperature of the catalyst was kept at 55°C. After 1 hour from the start of the reaction, the exit gas was analyzed with gas chromatography. The results are shown in Table 3.

The compositions of the catalysts prepared in this Example and also in Examples 15-24 are summarized in Table 4.

Example 15

In the same manner as in Example 14 but drying the alumina at 600°C and treating the alumina with hydrogen chloride gas at 500°C, the catalyst was prepared and the reaction was carried out. The results are shown in Table 3.

Example 16

In the same manner as in Example 14 but drying the alumina at 800°C and treating the alumina with hydrogen chloride gas at 700°C, the catalyst was prepared and the reaction was carried out. The results are shown in Table 3.

Example 17

In the same manner as in Example 14 but reacting R-21 and TFE at 100°C, the catalyst was prepared and the reaction was carried out. The results are shown in Table 3.

Example 18

In the same manner as in Example 15 but reacting R-21 and TFE at 100°C, the reaction was carried out. The results are shown in Table 3.

Example 19

In the same manner as in Example 15 but flowing R-21 and TFE at flow rates of 10 ml/min. and 10 ml/min., respectively (a molar ratio of 1:1), the reaction was carried out. The results are shown in Table 3.

Example 20

In the same manner as in Example 15 but flowing R-21 and TFE at flow rates of 10 ml/min. and 100 ml/min., respectively (a molar ratio of 1:10), the reaction was carried out. The results are shown in Table 3.

Example 21

In the same manner as in Example 15 but reacting R-21 and TFE at 150°C, the reaction was carried out. The results are shown in Table 3.

Example 22

In the same manner as in Example 15 but using Neobead GB (manufactured by Mizusawa Chemical Co., Ltd.) in place of activated alumina, the catalyst was prepared and the reaction was carried out. The results are shown in Table 3.

Example 23

In the same manner as in Example 15 but flowing R-21 and TFE at flow rates of 10 ml/min. and 30 ml/min., respectively (a molar ratio of 1:3), the reaction was carried out. The results are shown in Table 3.

Example 24

In the same manner as in Example 15 but treating the activated alumina with a mixture of hydrogen chloride (100 ml/min.) and R-21 (100 ml/min.), the catalyst was prepared and the reaction was carried out. The results are shown in Table 3.

Comparative Example 1

In a silver-lined autoclave, anhydrous aluminum chloride (5 g) was charged. The autoclave was cooled in a dry ice-acetone bath and its internal gas was evacuated. Then, R-21 (52 g, 0.5 mole) and TFE (50 g, 0.5 mole) were distilled and charged in the autoclave. After closing the autoclave, the interior mixture was heated while stirring at 100°C for 10 hours. The autoclave was opened and the reaction mixture was recovered and washed with water and hydrochloric acid to obtain a mixture of R-225ca and R-225cb (47 g, 0.23 mole). An yield was 46.3 % based on the amount of R-21. The results of gas chromatography are shown in Table 1.

Table 1

| Exam-ple No. | Alumina | Compound treating alumina | Reac-tion temp (°C) | Molar ratio of R-21/TFE | Conver-sion of R-21 (%) | Selectivities (%) | | | |
|---|---|---|---|---|---|---|---|---|---|
| | | | | | | R-22 + R-23 | R-225ca + R-225cb | $CHCl_3$ | R-224 |
| 1 | Neobead GB | R-21 | 30 | 1/3 | 99.8 | 3.5 | 92 | 3.5 | 1 |
| 2 | Neobead C | R-22 | 30 | 1/3 | 99.8 | 6 | 88 | 4 | 2 |
| 3 | Neobead GB | R-12 | 30 | 1/3 | 98.5 | 3.5 | 92 | 3 | 1.5 |
| 4 | Neobead GB | R-114 | 30 | 1/3 | 96.5 | 5 | 89 | 3 | 3 |
| 5 | Neobead GB | $CCl_4$ | 30 | 1/3 | 99.0 | 5 | 92 | 0.2 | 2.8 |
| 6 | NHA 3 | R-21 | 30 | 1/3 | 99.8 | 2.5 | 95 | 1 | 1.5 |
| 7 | KHA | R-21 | 30 | 1/3 | 99.8 | 10 | 78 | 10 | 2 |
| 8 | Neobead GB | R-21 | -20 | 1/3 | 99.8 | 3 | 93 | 1 | 3 |
| 9 | Neobead GB | R-21 | 80 | 1/3 | 99.8 | 10 | 85 | 4.5 | 0.5 |
| 10 | Neobead GB | R-21 | 30 | 1/10 | 99.8 | 3 | 93 | 3 | 1 |
| 11 | Neobead GB | R-21 | 30 | 1/1 | 99.5 | 27.5 | 63 | 7 | 2.5 |
| 12 | Neobead GB | R-21 | 160 | 1/3 | 99.7 | 9 | 64 | 25 | 2 |
| 13 | Neobead GB | R-21 | 200 | 1/3 | 99.5 | 9.5 | 52.5 | 31 | 7 |
| Com.1 | $AlCl_3$ as a catalyst | | 100 | 1/1 | 98.0 | --- | 48 | 44 | 8 |

EP 0 421 322 B1

Table 2

| Example No. | Composition of catalyst (wt.%) | | | |
|:---:|:---:|:---:|:---:|:---:|
| | Al | Cl | F | O |
| 1 | 45.5 | 2.3 | 20.1 | 32.1 |
| 2 | 50.5 | 0.9 | 6.4 | 42.2 |
| 3 | 49.2 | 0.7 | 11.4 | 38.7 |
| 4 | 49.1 | 0.5 | 10.5 | 39.9 |
| 5 | 51.5 | 3.5 | 0 | 45.0 |
| 6 | 45.8 | 2.4 | 20.3 | 31.5 |
| 7 | 45.7 | 2.2 | 20.2 | 31.9 |
| 8 | 45.5 | 2.3 | 20.1 | 32.1 |
| 9 | 45.5 | 2.3 | 20.1 | 32.1 |
| 10 | 45.5 | 2.3 | 20.1 | 32.1 |
| 11 | 45.5 | 2.3 | 20.1 | 32.1 |
| 12 | 45.5 | 2.3 | 20.1 | 32.1 |
| 13 | 45.5 | 2.3 | 20.1 | 32.1 |

Table 3

| Exam- ple No. | Alumina | Drying temp. (°C) | Treating temp. w/ HCl (°C) | Reac- tion temp. (°C) | Molar ratio of R-21/TFE | Conver- sion of R-21 (°C) | Selectivities (%) | | | |
|---|---|---|---|---|---|---|---|---|---|---|
| | | | | | | | R-22+ R-23 | R-225ca+ R-225cb | $CHCl_3$ | R-224 |
| 14 | NKH 3 | 400 | 300 | 55 | 1/6 | 42.1 | 9.1 | 80.4 | 5.3 | 5.2 |
| 15 | NKH 3 | 600 | 500 | 55 | 1/6 | 94.0 | 9.0 | 79.6 | 5.3 | 6.0 |
| 16 | NKH 3 | 800 | 700 | 55 | 1/6 | 97.2 | 15.3 | 66.6 | 11.5 | 6.5 |
| 17 | NKH 3 | 400 | 300 | 100 | 1/6 | 76.3 | 6.7 | 83.2 | 7.9 | 2.2 |
| 18 | NKH 3 | 600 | 500 | 100 | 1/6 | 100 | 14.5 | 65.6 | 14.0 | 5.9 |
| 19 | NKH 3 | 600 | 500 | 55 | 1/1 | 20.5 | 10.4 | 58.3 | 26.5 | 4.8 |
| 20 | NKH 3 | 600 | 500 | 55 | 1/10 | 100 | 5.3 | 86.5 | 1.1 | 7.1 |
| 21 | NKH 3 | 600 | 500 | 150 | 1/6 | 100 | 12.3 | 57.7 | 24.6 | 5.3 |
| 22 | Neobead GB | 600 | 500 | 55 | 1/6 | 93.9 | 11.2 | 75.5 | 5.6 | 7.7 |
| 23 | Neobead GB | 600 | 500 | 55 | 1/3 | 72.4 | 14.5 | 65.6 | 14.0 | 5.9 |
| 24 | NKH 3 | 600 | 500 | 55 | 1/3 | 95.8 | 5.1 | 92 | 0.2 | 2.7 |
| Com.1 | $AlCl_3$ as a catalyst | | | 100 | 1/1 | 98.0 | ---- | 48 | 44 | 8 |

EP 0 421 322 B1

Table 4

| Example No. | Composition of catalyst (wt.%) | | | |
|---|---|---|---|---|
| | Al | Cl | F | O |
| 14 & 17 | 50.6 | 5.7 | ---- | 43.7 |
| 15, 18, 19 20 & 21 | 51.8 | 2.7 | ---- | 45.5 |
| 16 | 52.0 | 2.2 | ---- | 45.8 |
| 22 & 23 | 50.4 | 6.2 | ---- | 43.4 |
| 24 | 45.6 | 2.6 | 20.1 | 31.7 |

Example 25

In a reactor tube having an inner diameter of 20 mm made of stainless steel SUS 316, anhydrous aluminum chloride particles (40 ml) were filled. After purging the internal atmosphere with dry nitrogen and adjusted the internal temperature at 0°C, R-21 and TFE were flowed at flow rates of 20 ml/min. and 60 ml/min., respectively (a molar ratio of 1:3). After 3 hours, the exit gas was analyzed with gas chromatography. The results are shown in Table 5.

Example 26

In the same manner as in Example 25 but changing the reaction temperature from 0°C to 50°C, the reaction was carried out. The results are shown in Table 5.

Example 27

In the same manner as in Example 25 but changing the reaction temperature from 0°C to -20°C, the reaction was carried out. The results are shown in Table 5.

Example 28

In the same manner as in Example 25 but changing the molar ratio of R-21 to TFE from 1:3 to 1:10, the reaction was carried out. The results are shown in Table 5.

Comparative Example 2

In the same manner as in Example 25 but changing the reaction temperature from 0°C to 110°C, the reaction was carried out. The results are shown in Table 5.

Comparative Example 3

In the same manner as in Example 25 but changing the reaction temperature from 0°C to 150°C, the reaction was carried out. The results are shown in Table 5.

Comparative Example 4

In the same manner as in Example 25 but changing the molar ration of R-21 to TFE from 1:3 to 1:1, the reaction was carried out. The results are shown in Table 5.

Table 5

| Example No. | Reaction temp. (°C) | Molar ratio of R-21/TFE | Conversion of R-21 (%) | Selectivities (%) | | | |
|---|---|---|---|---|---|---|---|
| | | | | R-22 + R-23 | R-225ca + R-225cb | CHCl3 | R-224 |
| 25 | 0 | 1/3 | 99.5 | 4 | 93 | 3 | 1 |
| 26 | 50 | 1/3 | 99.8 | 5 | 90 | 4.5 | 0.5 |
| 27 | -20 | 1/3 | 99.0 | 3.5 | 93 | 1 | 2.5 |
| 28 | 0 | 1/10 | 99.7 | 2.5 | 93.5 | 3 | 1 |
| Com.2 | 110 | 1/3 | 99.8 | 4 | 64 | 30 | 6 |
| Com.3 | 150 | 1/3 | 99.8 | 5 | 51 | 43 | 1 |
| Com.4 | 0 | 1.1 | 99.2 | 30 | 60 | 7 | 3 |

## Claims

1. A process for preparing 1,1,1,2,2-pentafluoro-3,3-dichloropropane and 1,1,2,2,3-pentafluoro-1,3-dichloropropane which comprises reacting tetrafluoroethylene with fluorodichloromethane in the presence of a catalyst selected from the group consisting of an aluminum chlorofluoride of the formula:

$$AlCl_xF_yO_z$$

wherein x, y and z are numbers which satisfy the equations:

$$x + y + 2z = 3, 0 < x < 3, 0 \leqq y < 3 \text{ and } 0 < z < 3/2$$

and anhydrous aluminum chloride provided that when the catalyst is anhydrous aluminum chloride, at least 2 moles of tetrafluoroethylene is reacted with one mole of fluorodichloromethane at a temperature of -20°C to +100°C.

2. The process according to claim 1, a molar ratio of fluorodichloromethane to tetrafluoroethylene is from 1:2 to 1:10.

3. The process according to claim 1, wherein a reaction is carried out in a gas phase.

4. The process according to claim 1, wherein said aluminum chlorofluoride is used as a catalyst and a reaction temperature is from -20 to +150°C.

5. The process according to claim 1, wherein anhydrous aluminum chloride is used as a catalyst and a reaction temperature is from -20 to +50°C.


**Patentansprüche**

1. Verfahren zur Herstellung von 1,1,1,2,2-Pentafluor-3,3-dichlorpropan und 1,1,2,2,3-Pentafluor-1,3-dichlorpropan, umfassend die Reaktion von Tetrafluorethylen mit Fluordichlormethan in der Gegenwart eines Katalysators, ausgewählt aus der Gruppe, bestehend aus einem Aluminiumchlorfluorid der Formel:

$$AlCl_xF_yO_z$$

worin x, y und z Zahlen sind, die die folgenden Gleichungen erfüllen:

$$x + y + 2z = 3, 0 < x < 3, 0 \leqq y < 3 \text{ und } 0 < z < 3/2$$

und wasserfreiem Aluminiumchlorid, mit dem Vorbehalt, daß dann, wenn der Katalysator wasserfreies Aluminiumchlorid ist, zumindest 2 Mol Tetrafluorethylen mit einem Mol Fluordichlormethan bei einer Temperatur von -20°C bis +100°C zur Reaktion gebracht werden.

2. Verfahren nach Anspruch 1, dadurch **gekennzeichnet**, daß ein molares Verhältnis von Fluordichlormethan zu Tetrafluorethylen von 1:2 bis 1:10 ist.

3. Verfahren nach Anspruch 1, dadurch **gekennzeichnet**, daß die Reaktion in einer Gasphase durchgeführt wird.

4. Verfahren nach Anspruch 1, dadurch **gekennzeichnet**, daß das Aluminiumchlorfluorid als ein Katalysator verwendet wird, und daß die Reaktionstemperatur von -20 bis + 150°C ist.

5. Verfahren nach Anspruch 1, dadurch **gekennzeichnet**, daß wasserfreies Aluminiumchlorid als ein Katalysator verwendet wird und daß die Reaktionstemperatur von -20 bis +50°C ist.


**Revendications**

1. Un procédé de préparation du 1,1,1,2,2-pentafluoro-3,3-dichloropropane et du 1,1,2,2,3-pentafluoro-1,3-dichloropropane qui comprend la réaction du tétrafluoroéthylène avec le fluorodichlorométhane en présence d'un catalyseur choisi dans le groupe constitué d'un chlorofluorure d'aluminium de formule:

$$AlCl_xF_yO_z$$

dans laquelle x, y et z sont des nombres qui satisfont aux équations :

$$x + y + 2z = 3, 0 < x < 3, 0 \leqq y < 3 \text{ et } 0 < z < 3/2$$

et du chlorure d'aluminium anhydre à la condition que, lorsque le catalyseur est du chlorure d'aluminium anhydre, au moins 2 moles de tétrafluoroéthylène sont mises à réagir avec 1 mole de fluorodichlorométhane à une température de -20°C à +100°C.

2. Le procédé selon la revendication 1, selon laquelle le rapport molaire entre le fluorodichlorométhane et le tétrafluoroéthylène est de 1:2 à 1:10.

3. Le procédé selon la revendication 1, selon laquelle la réaction est conduite en phase gazeuse.

4. Le procédé selon la revendication 1, selon laquelle ledit chlorofluorure d'aluminium est utilisé comme catalyseur et la température de réaction est de -20 à +150°C.

5. Le procédé selon la revendication 1, selon laquelle le chlorure d'aluminium anhydre est utilisé comme catalyseur et la température de réaction est de -20 à +50°C.